(19) Europäisches Patentamt / European Patent Office / Office européen des brevets

(11) **EP 3 054 282 B1**

(12) **FASCICULE DE BREVET EUROPEEN**

(45) Date de publication et mention de la délivrance du brevet:
**19.08.2020 Bulletin 2020/34**

(51) Int Cl.:
**G01N 21/47** *(2006.01)* **G01N 21/49** *(2006.01)*

(21) Numéro de dépôt: **16154146.1**

(22) Date de dépôt: **03.02.2016**

(54) **PROCÉDÉ DE CARACTÉRISATION D'UN ÉCHANTILLON PAR MESURE D'UN SIGNAL OPTIQUE RÉTRODIFFUSÉ**

VERFAHREN ZUR CHARAKTERISIERUNG EINER PROBE DURCH MESSUNG EINES ZURÜCKGESTREUTEN OPTISCHEN SIGNALS

METHOD FOR CHARACTERISING A SAMPLE BY MEASURING A BACKSCATTERED OPTICAL SIGNAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.02.2015 FR 1550982**

(43) Date de publication de la demande:
**10.08.2016 Bulletin 2016/32**

(73) Titulaire: **Commissariat à l'Énergie Atomique et aux Énergies Alternatives**
**75015 Paris (FR)**

(72) Inventeurs:
• **ROIG, Blandine**
**56160 GUEMENE SUR SCORFF (FR)**
• **KOENIG, Anne**
**38410 Saint Martin d'Uriage (FR)**
• **DINTEN, Jean-Marc**
**69008 LYON (FR)**
• **PERRAUT, François**
**38134 Saint Joseph de Riviere (FR)**

(74) Mandataire: **GIE Innovation Competence Group**
**310, avenue Berthelot**
**69372 Lyon Cedex 08 (FR)**

(56) Documents cités:
EP-A2- 2 762 064       WO-A1-2011/115627
US-A1- 2003 191 398    US-A1- 2012 302 892

## Description

### Domaine technique

L'invention se situe dans le domaine de la caractérisation des échantillons, et notamment des échantillons biologiques, et plus particulièrement la peau.

### Description de l'art antérieur

Les mesures optiques permettant de caractériser des échantillons sont répandues. Il s'agit en particulier de caractériser les propriétés optiques, ou la nature des matériaux qui composent un échantillon. On peut notamment citer les mesures basées sur la détection d'un signal rétrodiffusé par un échantillon illuminé par un faisceau lumineux. Il s'agit en particulier de la spectrométrie Raman, l'imagerie de fluorescence ou la spectrométrie de réflectance diffuse.

La demande US2003/191398 décrit par exemple un dispositif permettant l'obtention de spectres Raman à des fins de diagnostic. La demande US2012/0302892 décrit une association de différentes méthodes, par exemple la spectrométrie Raman, la fluorescence ou des mesures de réflectance. On peut également citer le document WO2011/115627, décrivant un procédé visant à estimer une taille caractéristique de diffuseurs dans un échantillon en faisant varier l'angle entre le faisceau lumineux illuminant l'échantillon et un signal rétrodiffusé par l'échantillon.

La spectrométrie de réflectance diffuse, souvent désignée par l'acronyme anglais DRS (Diffuse Reflectance Spectroscopy), consiste à exploiter la lumière rétrodiffusée par un objet diffusant soumis à un éclairement, généralement ponctuel. Cette technique s'avère performante pour caractériser des propriétés optiques d'objets, en particulier les propriétés de diffusion ou d'absorption.

Mise en œuvre sur la peau, cette technique permet par exemple de caractériser le derme, comme décrit dans le document EP 2762064. Les auteurs de ce document décrivent une sonde de mesure destinée à être appliquée contre la peau. Cette sonde comporte une fibre optique centrale, dite fibre d'excitation, destinée à diriger un faisceau lumineux vers un échantillon de peau. Des fibres optiques, disposées autour de la fibre centrale, dites fibres de détection, recueillent un signal optique rétrodiffusé par le derme. Des moyens d'analyse spectrale du signal optique ainsi collecté, couplés à des algorithmes de calcul, permettent d'estimer des paramètres du derme, en particulier la concentration de certains chromophores, par exemple l'oxyhémoglobine ou la déoxyhémoglobine, mais aussi des paramètres gouvernant le parcours des photons dans le derme, notamment le coefficient de diffusion réduit $\mu s'$ ainsi que le coefficient d'absorption $\mu a$.

Selon les principes de la diffusion de la lumière dans un milieu diffusant, plus l'écartement entre la fibre d'excitation et une fibre de détection est important, plus la lumière recueillie par la fibre de détection est fonction des propriétés optiques dans les couches profondes du derme, c'est-à-dire au-delà d'une plage de profondeur comprise entre 300 $\mu m$ et quelques mm. Autrement dit, plus la distance entre la fibre d'excitation et la fibre de détection augmente, plus le résultat est représentatif des propriétés optiques des couches profondes du milieu examiné. A l'inverse, plus cette distance diminue, plus le résultat est représentatif des couches superficielles du milieu diffusant.

Or, afin d'effectuer une mesure suffisamment sensible, chaque fibre a un diamètre égal à quelques centaines de microns, typiquement 500 $\mu m$ pour la fibre d'excitation et 300 $\mu m$ pour chaque fibre de détection. Par ailleurs, du fait de contraintes de fabrication, il est difficilement envisageable de réduire significativement la distance entre ces fibres. Il en résulte que l'écartement minimal entre chaque fibre ne peut pas être inférieur à une valeur seuil, supérieure à 400 $\mu m$, et le plus souvent millimétrique.

De ce fait, alors que le procédé et le dispositif décrits dans cette publication sont adaptés à la caractérisation du derme, ils ne sont pas aptes à caractériser l'épiderme, ce dernier s'étendant, selon les individus et les zones corporelles, sur les premières dizaines micromètres de la peau. L'invention vise à résoudre ce problème, en proposant un procédé et un dispositif de mesure aptes à déterminer les propriétés optiques l'épiderme et, de façon plus générale, les propriétés optiques de la couche superficielle d'un milieu diffusant.

### Description de l'invention

Ainsi, un objet de l'invention est un procédé de détermination d'une propriété optique d'un échantillon, comportant les étapes suivantes :

- illumination d'une surface de l'échantillon à l'aide d'un faisceau lumineux, de façon à former, à la surface dudit échantillon, une zone élémentaire d'illumination, correspondant à la partie de ladite surface éclairée sur ledit échantillon,
- détection de N signaux optiques, rétrodiffusés par l'échantillon, chaque signal optique émanant de la surface de l'échantillon, à une distance, dite distance de rétrodiffusion, de ladite zone élémentaire d'illumination, de manière à former autant de signaux détectés, N étant un entier supérieur à 1, chaque zone élémentaire de détection étant séparée de ladite zone élémentaire d'illumination ;

- détermination d'au moins une propriété optique de l'échantillon, par comparaison entre :

    - une fonction de chaque signal ainsi détecté,
    - et une pluralité d'estimations de ladite fonction chaque estimation étant réalisée en considérant une valeur prédéterminée de ladite propriété optique,

le procédé étant caractérisé en ce que

- lors de ladite étape de détection, au moins une distance de rétrodiffusion, séparant une zone élémentaire de détection de ladite zone élémentaire d'illumination, est inférieure à 200 μm.

Ainsi, chaque zone élémentaire de détection est distincte de la zone élémentaire d'illumination et ne superpose pas à cette dernière. Elle est séparée de cette dernière par une distance non nulle.

De préférence, on forme au moins autant de signaux détectés que de distances de rétrodiffusion différentes.

De préférence, au moins une distance de rétrodiffusion peut être inférieure à 150 μm.

De préférence, au moins deux distances de rétrodiffusion sont inférieures à 200 μm.

Ladite propriété optique peut être choisie parmi

- un facteur d'anisotropie, représentant un angle moyen de diffusion,
- un coefficient d'absorption,
- un coefficient de diffusion,
- un coefficient de diffusion réduit.

Le procédé peut comprendre également :

- la détection d'au moins deux signaux optiques rétrodiffusés émanant de la surface de l'échantillon :

    - un signal rétrodiffusé proche, émis à une distance de rétrodiffusion dite proche, inférieure à 200 μm,
    - un signal rétrodiffusé éloigné, émis à une distance de rétrodiffusion dite éloignée, supérieure à ladite distance proche,

- la détermination d'une première propriété optique à partir dudit signal proche,
- la détermination d'une deuxième propriété optique, différente de la première propriété optique, à partir dudit signal rétrodiffusé éloigné.

Il peut également comprendre :

- la détection d'au moins deux signaux optiques rétrodiffusés proches, émanant de la surface de l'échantillon à deux distances de rétrodiffusion différentes, ces deux distances étant inférieures à 200 μm,
- la détermination d'un coefficient de diffusion réduit à partir dudit signal optique éloigné,
- puis la détermination d'un coefficient de diffusion et d'un coefficient d'anisotropie de diffusion à partir desdits deux signaux optiques rétrodiffusés proches, ainsi que du coefficient de diffusion réduit préalablement déterminé.

Il peut comprendre :

- la détection d'au moins deux signaux optiques rétrodiffusés éloignés, émanant de la surface de l'échantillon à deux distances de rétrodiffusion différentes, les deux distances étant supérieures à 200 μm,
- la détermination d'un coefficient d'absorption à partir desdits signaux optiques rétrodiffusés éloignés.

L'échantillon examiné peut être la peau d'un humain ou d'un animal.

Le signal optique rétrodiffusé peut être mesuré à une pluralité de longueurs d'ondes.

Selon un mode de réalisation, la dite fonction du signal est la réflectance, représentant l'intensité du signal mesuré, correspondant à ladite distance de rétrodiffusion, relativement à l'intensité dudit faisceau lumineux.

<u>Description des figures.</u>

Les figures 1 et 2 représentent un premier mode de réalisation d'un dispositif selon l'invention.

Les figures 3A et 3B représentent respectivement un schéma du dispositif selon l'art antérieur et selon l'invention.

La figure 4 est un schéma représentant le parcours de la lumière dans un échantillon diffusant en fonction de la distance de rétrodiffusion.

Les figures 5A à 5C représentent des logigrammes de 3 procédés permettant la détermination d'au moins une propriété optique d'un échantillon.

La figure 6 représente une modélisation de la réflectance, à une distance de rétrodiffusion donnée, pour différentes valeurs du coefficient d'absorption $\mu_a$ et du coefficient de diffusion réduit $\mu_s'$.

Les figures 7A, 7B, 7C et 7D représentent une modélisation de la réflectance, pour quatre distances de rétrodiffusion respectives, la réflectance étant estimée pour différents couples de valeurs du coefficient de d'absorption $\mu_a$, du coefficient de diffusion réduit $\mu_s'$ et du coefficient d'anisotropie de diffusion g.

## Description détaillée

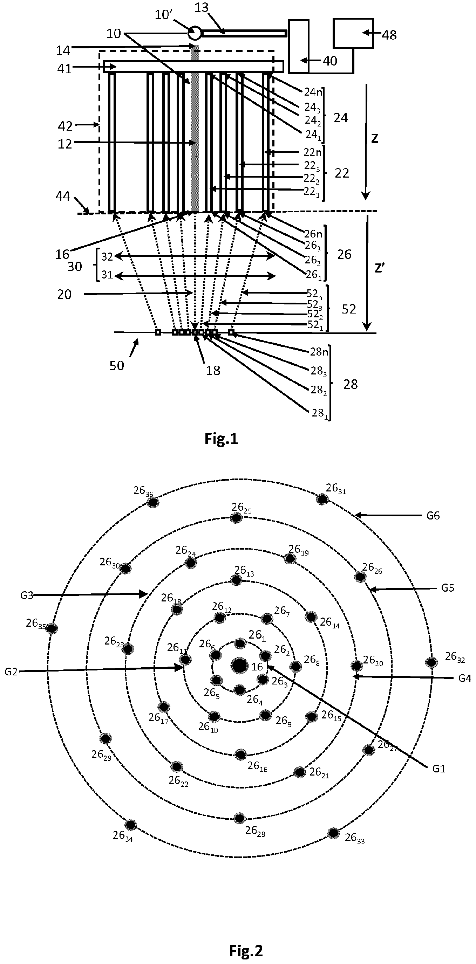

La figure 1 représente un premier mode de réalisation d'un dispositif selon l'invention. Il comporte une source de lumière 10, qui comprend dans ce premier exemple une source de lumière blanche 10'.

La source de lumière 10 comprend, dans ce premier mode de réalisation, une fibre optique d'illumination 12, s'étendant entre une extrémité proximale 14 et une extrémité distale 16. La fibre optique d'illumination 12 est apte à collecter la lumière, par une extrémité proximale 14 et à émettre un faisceau lumineux 20 vers l'échantillon par une extrémité distale 16, ledit faisceau lumineux étant alors dirigé vers la surface d'un échantillon 50. Dans une telle configuration, la source de lumière 10 est dite fibrée.

Le diamètre de la fibre optique d'émission 12 est compris entre 100 $\mu$m et 1 mm, et est par exemple égal à 400 $\mu$m.

Par source ponctuelle, on entend une source dont l'aire est inférieure à 1 cm$^2$, et de préférence inférieure à 5 mm$^2$, et encore de préférence inférieure à 1 mm$^2$.

Le dispositif comporte également une pluralité de fibres optiques de détection $22_1$, $22_2$, $22_3$...$22_n$, l'indice n étant compris entre 1 et N, N désignant le nombre de fibres optiques de détection dans le dispositif. N est un entier naturel compris généralement compris entre 1 et 100, et préférentiellement compris entre 5 et 50. Chaque fibre de détection $22_1$, $22_2$, $22_3$...$22_n$ s'étend entre une extrémité proximale $24_1$, $24_2$, $24_3$...$24_n$ et une extrémité distale $26_1$, $26_2$, $26_3$...$26_n$.

Sur la figure 1, les références 22, 24 et 26 désignent respectivement l'ensemble des fibres de détection, l'ensemble des extrémités proximales des fibres de détection et l'ensemble des extrémités distales des fibres de détection.

Le diamètre de chaque fibre optique de détection 22 est compris entre 50 $\mu$m et 1 mm, et est par exemple égal à 300 $\mu$m.

L'extrémité proximale 24 de chaque fibre optique de détection 22 est apte à être optiquement couplée à un photodétecteur 40.

L'extrémité distale $26_1$, $26_2$, $26_3$...$26_n$ de chaque fibre optique de détection 22 est apte à collecter respectivement un signal optique $52_1$, $52_2$, $52_3$...$52_n$, rétrodiffusé par l'échantillon 50, lorsque ce dernier est exposé au faisceau lumineux 20.

Le photodétecteur 40 est apte à détecter chaque signal optique $52_1$, $52_2$, $52_3$...$52_n$ de façon à former un signal $(S_1, S_2, S_3, ...S_n)$ en fonction de chaque signal optique détecté.

Il peut s'agir d'un spectrophotomètre, apte à établir le spectre en longueur d'onde du signal optique collecté par la fibre optique de détection 22 auquel il est couplé.

Le photodétecteur est apte à être connecté à un microprocesseur 48, ce dernier étant configuré pour mettre en œuvre les procédés décrits ci-après.

Les fibres optiques de détection 22 s'étendent parallèlement les unes aux autres, parallèlement à un axe longitudinal autour de la fibre optique d'émission 12. Elles sont maintenues fixes les unes par rapport aux autres par un élément de maintien 42. Leurs extrémités distales 26 sont coplanaires, et définissent un plan de détection 44.

La figure 2 représente une vue en coupe du dispositif, dans le plan de détection, formé par l'ensemble des extrémités distales 26 des N fibres de détection. Dans cet exemple, N est égal à 36. Comme on peut le voir, les fibres optiques de détection sont réparties selon :

- un premier groupe $G_1$ de six fibres optiques de détection $22_1$...$22_6$, disposées régulièrement le long d'un cercle centré sur la fibre optique d'émission 12, de telle sorte que l'extrémité distale $26_1$...$26_6$ de chaque fibre de ce groupe soit distante de l'extrémité distale 16 de la fibre optique d'émission 12 d'une première distance $d_1$ égale à 300 $\mu$m.

- un deuxième groupe $G_2$ de six fibres optiques de détection $22_7$...$22_{12}$, disposées régulièrement le long d'un cercle centré sur la fibre optique d'émission 12, de telle sorte que l'extrémité distale $26_7$...$26_{12}$ de chaque fibre de ce groupe soit distante de l'extrémité distale 16 de la fibre optique d'émission 12 d'une deuxième distance $d_2$ égale à 700 $\mu$m.

- un troisième groupe $G_3$ de six fibres optiques de détection $22_{13}...22_{18}$, disposées régulièrement le long d'un cercle centré sur la fibre optique d'émission 12, de telle sorte que l'extrémité distale $26_{13}...26_{18}$ de chaque fibre de ce groupe soit distante de l'extrémité distale 16 de la fibre optique d'émission 12 d'une troisième distance $d_3$ égale à 1,1 mm.

- un quatrième groupe $G_4$ de six fibres optiques de détection $22_{19}...22_{24}$, disposées régulièrement le long d'un cercle centré sur la fibre optique d'émission 12, de telle sorte que l'extrémité distale $26_{19}...26_{24}$ de chaque fibre de ce groupe soit distante de l'extrémité distale 16 de la fibre optique d'émission 12 d'une quatrième distance $d_4$ égale à 1,5 mm.

- un cinquième groupe $G_5$ de six fibres optiques de détection $22_{25}...22_{30}$, disposées régulièrement le long d'un cercle centré sur la fibre optique d'émission 12, de telle sorte que l'extrémité distale $26_{25}...26_{30}$ de chaque fibre de ce groupe soit distante de l'extrémité distale 16 de la fibre optique d'émission 12 d'une cinquième distance $d_5$ égale à 2 mm.

- un sixième groupe $G_6$ de six fibres optiques de détection $22_{31}...22_{36}$, disposées régulièrement le long d'un cercle centré sur la fibre optique d'émission 12, de telle sorte que l'extrémité distale $26_{31}...26_{36}$ de chaque fibre de ce groupe soit distante de l'extrémité distale 16 de la fibre optique d'émission 12 d'une sixième distance $d_6$ égale à 2,5 $\mu$m.

Lorsqu'on évoque une distance entre deux fibres, ou entre une fibre ou un faisceau lumineux, on entend une distance centre à centre.

Ainsi, chaque extrémité distale $26_n$ d'une fibre optique de détection $22_n$ est placée, dans un plan perpendiculaire à l'axe longitudinal Z selon lequel s'étendent ces fibres, à une distance $d_n$ de la source de lumière 10 (c'est-à-dire de l'extrémité distale 16 de la fibre d'émission 12), et, par conséquent, à une distance $d_n$ du faisceau lumineux 20 dirigé vers l'échantillon 50.

Le dispositif peut comporter un photodétecteur 40, apte à être couplé à l'extrémité proximale 24n de chaque fibre optique de détection 22n. Dans cet exemple, le photodétecteur est un spectrophotomètre, apte à déterminer le spectre d'un signal optique $52_1...52_n$ rétrodiffusé par l'échantillon lorsque ce dernier est exposé au faisceau lumineux 20. Pour cela, les extrémités proximales 24 de chaque groupe de fibres optiques de détection, décrits ci-dessus, sont regroupées et sont, groupe par groupe, successivement couplées au photodétecteur 40 au moyen d'un commutateur optique 41. Ainsi, le photodétecteur permet de mesurer le spectre d'un rayonnement rétrodiffusé par l'échantillon, sous l'effet d'une illumination par le faisceau lumineux 20.

Le dispositif peut comporter une fibre optique 13, dite de retour d'excitation, dont une extrémité proximale est couplée à la source de lumière 10 et dont une extrémité distale est apte à être couplée au photodétecteur 40. L'utilisation de cette fibre optique est détaillée par la suite.

Le dispositif comporte également un système optique 30, présentant un facteur de grandissement G et un axe optique Z'. Dans cet exemple, l'axe optique Z' est confondu avec l'axe longitudinal Z selon lequel s'étendent les fibres optiques de détection, ce qui constitue une configuration préférée. Dans cet exemple, le système optique 30 comporte:

- un objectif de microscope 31, placé en configuration foyer infini, apte à produire une image à l'infini de la surface de l'échantillon observé, lorsque ce dernier est placé au foyer de cet objectif. Dans cet exemple, il s'agit d'un objectif de marque Zeiss, de référence Plan Aprochromat 20X, d'ouverture numérique 0.8, et de distance focale $f_1 = 8,25$ mm ;

- un doublet achromat 32, de distance focale $f_2 = 50$ mm, accolé à l'objectif 31, apte à projeter l'image fournie par ce dernier dans le plan de détection 44, ce plan étant placé à la distance focale du doublet.

Le système optique 30 décrit ci-dessus présente un facteur de grandissement G égal au rapport des distances focales, soit G ≈ 6. De préférence, le grandissement est supérieur à 2, voire supérieure à 5 ou à 10.

De préférence, le système optique 30 est amovible et interchangeable, ce qui permet, en utilisant le même dispositif, l'utilisation de systèmes optiques présentant des facteurs de grandissements différents.

D'une façon générale, le système optique 30 permet de former une image de la surface de l'échantillon 50 sur le plan de détection 44 formé par les extrémités distales 26 de chaque fibre optique de détection 22, avec un facteur de grandissement G donné. Ainsi, chaque extrémité distale $26_1$, $26_2...26_n$ est respectivement conjuguée à une zone élémentaire de détection $28_1$, $28_2...28_n$ de la surface de l'échantillon. De cette façon, chaque fibre optique de détection $22_1$, $22_2,...22_n$ est apte à collecter respectivement un signal optique élémentaire $52_1$, $52_2,...52_n$ rétrodiffusé par l'échan-

tillon, chaque signal optique élémentaire $52_1$, $52_2$,...$52_n$ émanant de la zone élémentaire de détection $28_1$, $28_2$...$28_n$.

Ainsi, chacune desdites extrémités distales $26_1$, $26_2$...$26_n$ peut être située dans un plan focal image du système optique 30, et conjuguée à une zone élémentaire de détection $28_1$, $28_2$...$28_n$ située dans le plan focal objet dudit système optique, à la surface de l'échantillon.

De même, l'extrémité distale 16 de la fibre d'émission 12 est conjuguée à une zone élémentaire d'illumination 18 à la surface de l'échantillon, cette zone élémentaire d'illumination constituant le point d'impact du faisceau lumineux 20 à la surface de l'échantillon 50.

D'une façon générale, et quel que soit le mode de réalisation, le terme zone élémentaire désigne une partie de la surface de l'échantillon dont les dimensions sont suffisamment faibles pour considérer qu'elle est traversée par un rayonnement lumineux homogène. Autrement dit, une zone élémentaire est une zone de forme délimitée, de préférence ponctuelle, c'est-à-dire dont le diamètre ou la diagonale est inférieure à 5 mm, et de préférence inférieure à 1 mm, voire inférieure à 500 $\mu$m.

Une zone élémentaire d'illumination 18 est traversée par le faisceau lumineux 20, se propageant en direction de l'échantillon 50, tandis qu'une zone élémentaire de détection 28n est traversée par un rayonnement rétrodiffusé (ou signal optique) 52n, ce rayonnement étant produit par la rétrodiffusion, dans l'échantillon, du faisceau lumineux 20. Le couplage optique, réalisé par le système optique 30, permet à chaque fibre de détection 22n de collecter le rayonnement rétrodiffusé élémentaire 52n, ce dernier correspondant au rayonnement rétrodiffusé traversant la zone élémentaire 28n.

L'élément de maintien 42 peut assurer une liaison rigide entre les fibres optiques de détection 22 et le système optique 30, de façon à maintenir le plan de détection 44, formé par les extrémités distales 26 des fibres optiques de détection à une distance fixe du système optique 30.

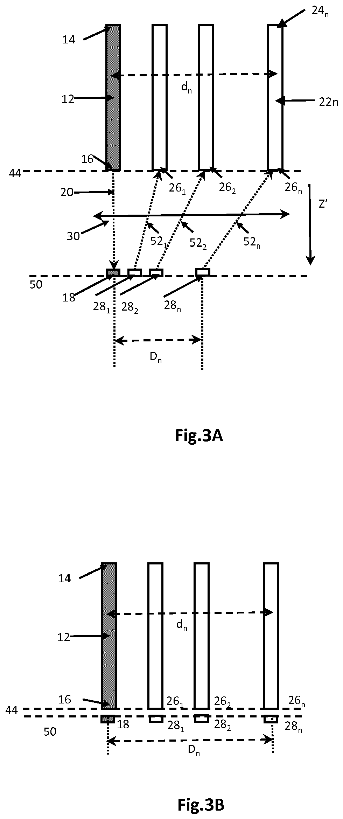

En lien avec la figure 3A, et cela constitue un des points essentiels de l'invention, si dn est la distance entre l'extrémité distale 26n d'une fibre de détection 22n et l'extrémité distale 16 de la fibre d'émission 12, distance calculée dans un plan perpendiculaire à l'axe optique Z', la distance Dn entre la zone élémentaire de détection 28n, conjuguée de ladite extrémité distale 26n, et la zone élémentaire d'illumination 18, conjuguée de ladite extrémité distale 16, est telle que :

$$Dn = \frac{dn}{G}$$

La distance $D_n$ est appelée distance de rétrodiffusion, car elle correspond à la distance, à partir de la zone élémentaire d'illumination 18, à laquelle émanent les photons rétrodiffusés. Cela correspond à la distance entre la zone élémentaire d'illumination 18 et une zone élémentaire de détection 28n.

Lorsque son facteur de grandissement est supérieur à 1, le système optique 30 tend à rapprocher significativement les zones élémentaires de détection 28n de la zone élémentaire d'illumination 18 par rapport à la configuration de l'art antérieur, dépourvue de système optique, et représentée sur la figure 3B dans laquelle les extrémités distales de chaque fibre optique (fibres optiques de détection et fibre optique d'illumination) sont placées au contact de la surface de l'échantillon. Dans cette dernière configuration, la distance de rétrodiffusion Dn est égale à la distance dn entre les extrémités distales respectives d'une fibre optique de détection 22n et de la fibre optique d'illumination 12.

En résumé, dans la configuration de l'art antérieur, *Dn = dn,* tandis qu'en mettant en œuvre le système optique 30, $Dn = \frac{dn}{G}$.

Si la distance entre le faisceau lumineux et l'extrémité distale d'une fibre optique de détection est, en amont du système optique, égale à une première distance, la distance de rétrodiffusion est à la surface de l'échantillon, égale à ladite première distance pondérée par l'inverse duditledit facteur de grandissement.

Le terme « en amont » se comprend en considérant la direction de propagation de la lumière.

Lorsque le facteur de grandissement est inférieur à 1, les zones élémentaires de détection 28n sont éloignées les unes des autres, et éloignées de la zone élémentaire d'illumination, leur surface étant augmentée du carré du facteur de grandissement, par rapport à la surface de l'extrémité distale à laquelle elles sont respectivement associées. Cela permet d'effectuer une caractérisation de l'échantillon avec une résolution spatiale plus faible, mais sur une profondeur plus élevée que selon l'art antérieur.

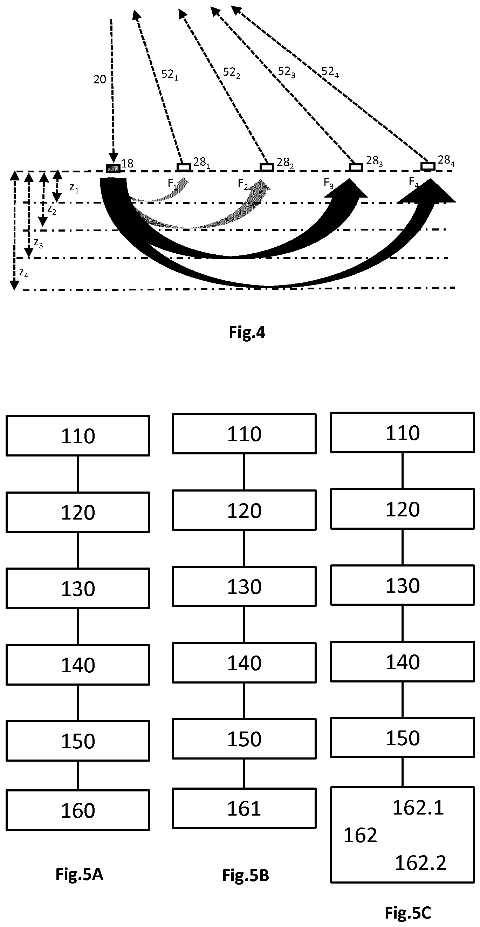

La figure 4 résume cet effet technique, chaque flèche $F_1$, $F_2$, $F_3$ et $F_4$ représentant le trajet, dans l'échantillon 50, des photons contribuant aux signaux rétrodiffusés respectifs $52_1$, $52_2$, $52_3$, $52_4$ respectivement associés aux zones élémentaires de détection $28_1$, $28_2$, $28_3$, $28_4$. Plus l'écartement entre les zones élémentaires de détection $28_1$, $28_2$, $28_3$, $28_4$ et la zone élémentaire d'illumination 18 est élevée, plus la profondeur examinée $z_1$, $z_2$, $z_3$, $z_4$ de l'échantillon est importante. Inversement, lorsque le facteur de grandissement est supérieur à 1, plus il est élevé, plus l'écartement entre les zones élémentaires de détection et la zone élémentaire d'illumination est faible plus la profondeur examinée de l'échantillon est faible.

Plus particulièrement, lorsque l'échantillon est de la peau, le fait de rapprocher les zones élémentaires de détection de la zone élémentaire d'illumination permet la caractérisation de l'épiderme, qui constitue la couche superficielle de la peau, s'étendant jusqu'à une profondeur comprise entre 100 et 300 $\mu$m, ce qui n'est pas envisageable avec les dispositifs de l'art antérieur, dont l'espacement entre chaque fibre est de l'ordre de quelques centaines de micromètres à quelques millimètres.

Autrement dit, le dispositif selon l'invention permet de détecter des signaux de rétrodiffusion émis par l'échantillon à des distances de rétrodiffusion significativement plus faibles que selon l'art antérieur, et cela permet une caractérisation des propriétés optiques de la couche superficielle de l'échantillon observé.

Lorsque le photodétecteur est un spectromètre, le dispositif permet d'effectuer des mesures du spectre rétrodiffusé selon des distances de rétrodiffusion de quelques dizaines à environ 200 $\mu$m. On parle de spectroscopie de microréflectance diffuse, par opposition à la spectroscopie de réflectance diffuse, connue de l'art antérieur.

On décrit à présent un procédé susceptible d'être mis en œuvre par le dispositif précédemment décrit, afin d'estimer une ou plusieurs propriétés optiques de l'échantillon examiné, et plus particulièrement d'une couche superficielle de cet échantillon, cette couche s'étendant en particulier entre la surface et une profondeur inférieure à $1/\mu_s'$.

Dans la plupart des tissus biologiques, et pour les longueurs d'onde du spectre visible ou proche infra-rouge, le coefficient de diffusion réduit est compris entre 10 cm$^{-1}$ et 70 cm$^{-1}$. Aussi, le procédé décrit ci-dessous est apte à caractériser une couche superficielle, s'étendant entre la surface de l'échantillon et une profondeur inférieure à 1 mm, et plus particulièrement inférieure à 500 $\mu$m, voire 200 $\mu$m ou 100 $\mu$m.

Une des applications visées est la caractérisation de la peau, laquelle comporte trois couches. La couche superficielle est l'épiderme, dont l'épaisseur varie selon les individus et la partie du corps, et est généralement comprise entre quelques dizaines de $\mu$m et 100 $\mu$m. Le coefficient de diffusion réduit de l'épiderme est compris entre 50 et 70 cm$^{-1}$, selon la longueur d'onde. Il est par exemple cité, dans la littérature, une valeur de 66 cm$^{-1}$ à la longueur d'onde de 500 nm.

Les deux autres couches de la peau, plus profondes que l'épiderme, et plus épaisses que ce dernier sont respectivement le derme, dont l'épaisseur varie entre 1 et 2 mm, puis l'hypoderme, dont l'épaisseur peut dépasser plusieurs mm.

Le terme propriété optique désigne notamment un facteur gouvernant l'absorption et la diffusion des photons dans le milieu diffusant, en particulier un coefficient d'absorption, un coefficient de diffusion, un coefficient de diffusion réduit, un coefficient d'anisotropie de la diffusion.

En lien avec la figure 5A, on décrit les principales étapes d'un procédé permettant l'estimation des propriétés optiques de diffusion, en particulier le coefficient d'absorption $\mu_a$ et le coefficient de diffusion réduit $\mu_s'$. Dans cet exemple, le facteur de grandissement G s'élève à 6. Comme représenté sur la figure 2, le dispositif comporte 36 fibres de détection, divisées en 6 groupes de 6 fibres, en fonction de la distance $d_n$ séparant chaque fibre de détection $22n$ du faisceau d'illumination 50. Ces distances $d_n$ s'élèvent respectivement à 300 $\mu$m ; 700 $\mu$m ; 1.1 mm ; 1.5 mm ; 2 mm ; 2.5 mm

Grâce au système optique 30, chaque fibre de détection $22n$ est conjuguée à une zone élémentaire de détection $28_n$. Les zones élémentaires de détection sont alors elles-mêmes divisées en 6 groupes, en fonction de la distance de rétrodiffusion $D_n$ séparant chaque zone élémentaire de détection $28_n$ à la zone élémentaire d'illumination 18. Compte tenu du facteur de grandissement, les distances $D_n$ s'élèvent respectivement à environ 50 $\mu$m ; 120 $\mu$m ; 185 $\mu$m ; 250 $\mu$m ; 333 $\mu$m ; 415 $\mu$m.

On procède alors selon les étapes suivantes :

1$^{\text{ère}}$ étape 110 : application du dispositif préalablement décrit, en regard de l'échantillon 50, de telle sorte que la surface examinée soit placée au foyer du système optique 30.

2$^{\text{ième}}$ étape 120 : illumination de l'échantillon en dirigeant un faisceau lumineux 20 contre la surface de l'échantillon, la partie de la surface illuminée constituant la zone élémentaire illuminée 18.

3$^{\text{ième}}$ étape 130 : collection d'un signal optique $52_1$, $52_2$, $52_3$, $52_4$... $52_n$ respectivement rétrodiffusé par l'échantillon, au niveau de chaque zone élémentaire de détection $28_1$, $28_2$, $28_3$, $28_4$... $28_n$, par la fibre optique $22_1$, $22_2$, $22_3$, $22_4$... $22_n$ de détection dont l'extrémité distale $26_1$, $26_2$, $26_3$, $26_4$... $26_n$ est respectivement conjuguée à ladite zone élémentaire $28_1$, $28_2$, $28_3$, $28_4$... $28_n$.

4$^{\text{ième}}$ étape 140 : mesure, à l'aide d'un photodétecteur 40, du signal $S_n$ représentatif du signal optique rétrodiffusé à chaque distance $D_n$ de la zone élémentaire d'illumination. Le signal $S_n$ peut être notamment établi en cumulant les signaux optiques collectés par les fibres optiques de détection d'un même groupe. Lorsque le détecteur est un détecteur spectrométrique, ce qui constitue le mode de réalisation préféré, il génère le spectre du signal $S_n$

5ième étape 150 : A l'aide de chaque signal $S_n$ correspondant à une distance de rétrodiffusion $D_n$, détermination d'une fonction $R_n$ appelée réflectance du signal, cette réflectance étant fonction du signal $S_n$ et de paramètres de calibration. Ainsi, $R_n = f_{calib}(S_n)$, ou $f_{calib}$ désigne une fonction de calibration, dépendant de l'instrumentation mise en œuvre, par exemple l'efficacité de collection par les fibres, la fonction de réponse du détecteur et l'intensité du faisceau lumineux incident. La fonction de calibration $f_{calib}$ peut être obtenue au cours d'une phase de calibration, préalablement ou postérieurement à la mesure sur l'échantillon.

Par exemple, la réflectance $R_n$ peut être obtenue, à partir de $S_n$, selon l'expression :

$$R_n = f_{calib}(S_n) = \frac{S_n - S_{ref}}{S_{source}}$$

ou selon l'expression

$$R_n = f_{calib}(S_n) = \frac{S_n - S_{ref}}{S_{source}} \times \frac{R_{std-n}}{S_{std-n}}$$

où :

- $S_n$ est le signal détecté correspondant à la distance $D_n$ de la zone élémentaire d'illumination ;
- $S_{ref}$ est un signal de calibration, traduisant les réflexions parasites du système optique, obtenu en activant la source de lumière, mais sans présence de l'échantillon, ce dernier étant par exemple remplacé par un écran absorbant de type écran noir ;
- $S_{source}$ est le signal produit par la source de lumière. Ssource peut notamment être établi en couplant la source de lumière au photodétecteur, par exemple au moyen d'une fibre optique dite de retour d'excitation 13, représentée sur la figure 1 ;
- $S_{std-n}$ est un signal de rétrodiffusion mesuré, en considérant une distance de rétrodiffusion $D_n$, sur un fantôme, dont les propriétés optiques (absorption, diffusion) sont connues, en utilisant le même dispositif que celui mis en œuvre pour acquérir le signal Sn ;
- $R_{std-n}$ est une estimation de la réflectance produite, par ledit fantôme, à la distance de rétrodiffusion $D_n$.

D'autres fonctions $f_{calib}$ établissant un lien entre le signal Sn mesuré par le photodétecteur et la réflectance Rn, notamment en prenant en compte les paramètres de l'instrumentation mise en œuvre, sont utilisables.

D'une façon générale, la réflectance représente l'intensité du signal rétrodiffusé. Elle est généralement normalisée par l'intensité du faisceau incident au détecteur, auquel cas elle représente une fraction du faisceau incident rétro-diffusée.

6ième étape 160 : pour au moins une longueur d'onde $\lambda$, et en considérant au moins deux distances $D_n$ de rétrodif-fusion, détermination du couple $(\mu_a(\lambda), \mu'_s(\lambda))$ présentant le moindre écart entre la réflectance mesurée $R_n(\lambda)$, à la longueur d'onde $\lambda$, et une réflectance modélisée $R_{n,\mu a,\mu s'}^{model}(\lambda)$ pour différentes valeurs de $\mu_a(\lambda)$ et de $\mu'_s(\lambda)$, à une distance de rétrodiffusion $D_n$. Cette détermination peut être réalisée par la minimisation d'un écart quadratique, et par exemple selon l'expression :

$$\left(\mu_a(\lambda), \mu'_s(\lambda)\right) = argmin_{\left(\mu_a(\lambda),\mu'_s(\lambda)\right)} \left(\sum_{n=1}^{N} \left(R_{n,\mu a,\mu s'}^{model}(\lambda) - R_n(\lambda)\right)^2\right)$$

où :

- N désigne le nombre de distances prises en compte ;
- $R_{n,\mu a,\mu s'}^{model}$ est une fonction de réflectance modélisée, établie pour la distance de rétrodiffusion $D_n$, et pour un couple de valeurs $\mu_a$ et $\mu_s'$.

Les différentes valeurs de réflectance modélisée $R_{n,\mu a,\mu s\prime}^{model}$ sont obtenues, pour une pluralité de couples de valeurs $\mu_a$, $\mu_s\prime$ au cours d'une phase de calibration, par simulation numérique, ou expérimentalement, sur des échantillons étalons dont les propriétés optiques sont connues.

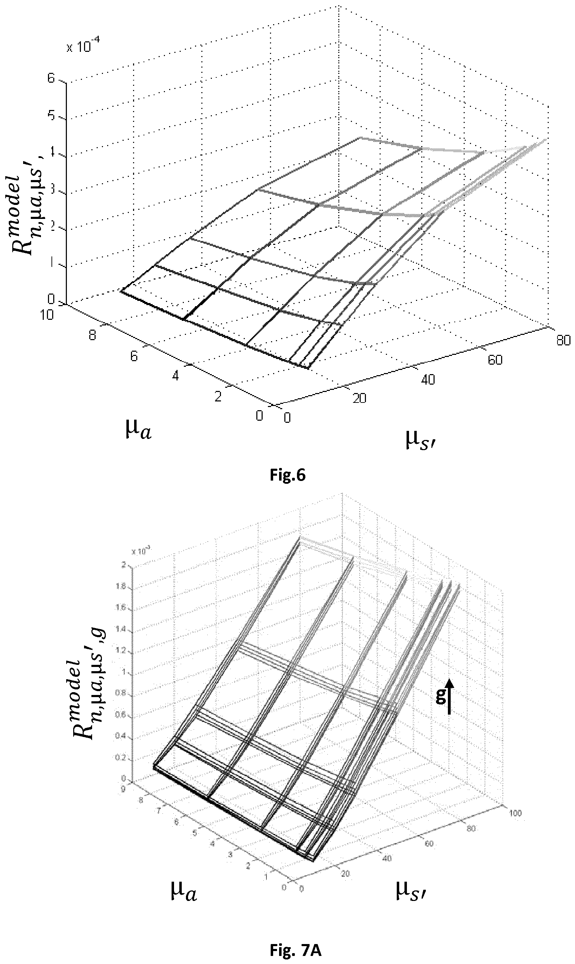

Pour une distance de rétrodiffusion $D_n$ donnée, on peut représenter une pluralité de réflectances $R_{n,\mu a,\mu s\prime}^{model}$ modélisées en fonction de $\mu a$ et de $\mu s\prime$. La figure 6 donne un exemple de représentation de telles réflectances modélisées, en considérant une distance de rétrodiffusion égale à 400 $\mu m$ et en prenant en compte des valeurs du coefficient d'absorption comprises entre 0 et 10 cm$^{-1}$, et des valeurs du coefficient de diffusion réduit comprises entre 0 et 100 cm$^{-1}$.

D'une façon générale, la notation $R_{n,p}^{model}$, désigne une réflectance modélisée à la distance de rétrodiffusion $D_n$, en prenant en compte des valeurs prédéterminées d'au moins un paramètre optique p. Le paramètre p peut correspondre à une propriété optique, ou un ensemble de propriétés optiques.

Les étapes 150 et 160 sont mises en œuvre par le microprocesseur 48, préalablement programmé à cet effet, et dont les données d'entrée sont les mesures réalisées par le photodétecteur 40.

Les inventeurs ont constaté que lorsque la distance de rétrodiffusion, séparant la zone d'illumination élémentaire d'une zone de détection élémentaire, est inférieure à 200 $\mu m$, ou, plus généralement, inférieure à un ratio $1/\mu s\prime$, le procédé décrit ci-dessus permet de caractériser les propriétés optiques de la couche superficielle de l'échantillon examiné, cette couche superficielle s'étendant entre la surface de l'échantillon et une profondeur inférieure à $1/\mu s\prime$.

Les figures 7A à 7D, réalisées par les inventeurs, représentent la valeur de la réflectance modélisée $R_{n,\mu a,\mu s\prime}^{model}$ en fonction des coefficients $\mu a$, $\mu s\prime$, en considérant un milieu semi-infini, et en considérant également un autre paramètre optique : le paramètre d'anisotropie de diffusion, noté g.

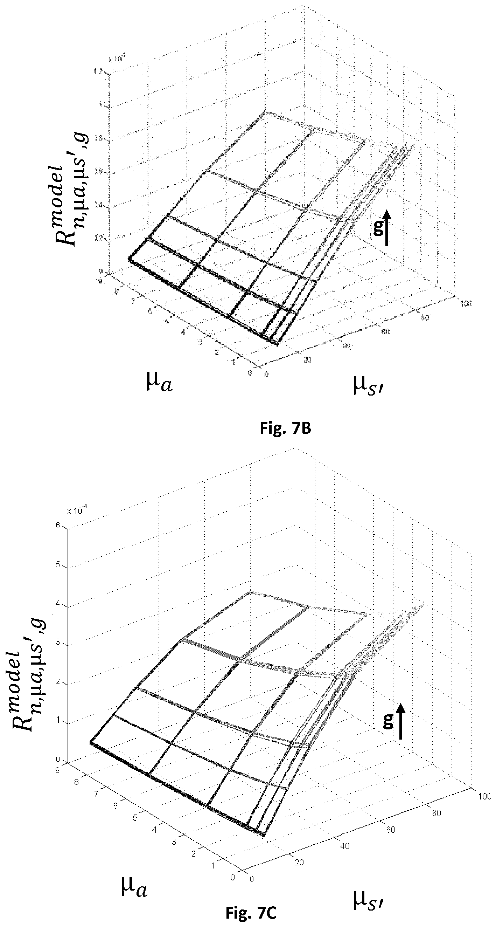

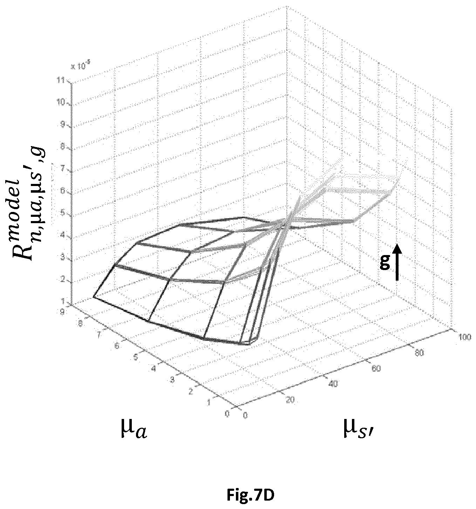

Ces simulations ont été réalisées avec un code de calcul modélisant le transport des photons de type Monte Carlo. La distance de rétrodiffusion est respectivement égale à 65 $\mu m$ pour la figure 7A, 120 $\mu m$ pour la figure 7B, 185 $\mu m$ pour la figure 7C et 415 $\mu m$ pour la figure 7D. Sur chaque figure, on a considéré quatre valeurs de g : 0,6 ; 0,7 ; 0,8 ; 0, 9.

Le facteur d'anisotropie de la diffusion, noté g, représente le cosinus moyen de l'angle de diffusion au cours d'une diffusion. Ce facteur, sans dimension, est défini dans la littérature par l'expression :

$$g = \int_{-1}^{1} f(\cos(\theta))\cos(\theta)d(\cos(\theta))$$

où

- $\theta$ représente l'angle de diffusion ;
- $f(\cos(\theta))$ est la probabilité associée à chaque valeur du cosinus de l'angle de diffusion.

Une diffusion parfaitement anisotrope, dans laquelle tous les angles de diffusion seraient équiprobables, se traduit par un coefficient d'anisotropie égal à 0. A l'inverse, en l'absence de diffusion, les photons ne sont pas déviés et le coefficient d'anisotropie est égal à 1. La propagation de la lumière dans le milieu est alors uniquement gouvernée par le coefficient d'absorption.

Dans les milieux biologiques, il est admis que la diffusion est anisotrope, privilégiant les faibles angles de diffusion. On parle de diffusion vers l'avant. Le facteur d'anisotropie est choisi de façon arbitraire, et généralement entre 0,8 et 0,95. Ce facteur est alors utilisé en tant que facteur de correction appliqué au coefficient de diffusion $\mu_s$ afin d'établir un coefficient de diffusion dit réduit, $\mu s\prime$, tel que :

$$\mu_s\prime = \mu_s\,(1\text{-}g)$$

$\mu_s\prime$ est un coefficient de diffusion réduit, prenant en compte la nature anisotrope de la diffusion dans le milieu diffusant.

Sur la figure 7A, on peut observer plusieurs nappes sensiblement parallèles les unes aux autres, chaque nappe correspondant à une valeur du coefficient d'anisotropie de diffusion g. Les différences entre les différentes nappes s'estompent peu à peu sur les figures 7B et 7C, et deviennent négligeables sur la figure 7D, sur laquelle on n'observe qu'une seule nappe, qui correspond à la superposition de quatre nappes respectivement affectées aux différentes valeurs de g.

Sur la base de la figure 7D, qui correspond à une distance de rétrodiffusion supérieure à quelques centaines de $\mu m$, l'influence de la valeur du coefficient d'anisotropie g sur la réflectance est faible : quelle que soit sa valeur, la valeur de la réflectance, pour un couple ($\mu$a, $\mu$s′) donné, n'évolue pas de façon significative. La diffusion de la lumière est correctement décrite par les coefficients $\mu$a et $\mu$s′ moyennant une détermination arbitraire du coefficient d'anisotropie.

Lorsque la distance de rétrodiffusion est faible, par exemple inférieure à 100 $\mu$m ou 200 $\mu$m, comme c'est le cas sur la figure 7A, et, dans une moindre mesure, sur les figures 7B et 7C, la réflectance évolue de façon non négligeable en fonction du coefficient d'anisotropie. Sur la figure 7A, qui correspond à une distance de rétrodiffusion de 65 $\mu$m, les différentes nappes, correspondant à différentes valeurs de g, se séparent distinctement. Aussi, une même valeur de réflectance peut correspondre à plusieurs couples ($\mu_a$, $\mu_s$′), selon la valeur du coefficient d'anisotropie g. Ainsi, la prise en compte d'un coefficient d'anisotropie défini de façon arbitraire n'est plus adaptée. Il devient alors utile de le déterminer expérimentalement.

Autrement dit, au voisinage de la surface de l'échantillon, la diffusion des photons ne peut plus être modélisée, de façon satisfaisante, en considérant le uniquement coefficient d'absorption $\mu_a$ et le coefficient de diffusion réduit $\mu_s$′, mais en considérant également le coefficient d'anisotropie de la diffusion.

Selon un deuxième exemple, représenté sur la figure 5B, et sous réserve de disposer d'un nombre suffisant de mesures à des distances de rétrodiffusion $D_n$ différentes et inférieures à 200 $\mu$m, on peut déterminer conjointement $\mu_a$, $\mu_s$′ et g, (ou $\mu_a$, $\mu_s$ et g) en appliquant les étapes 110 à 150 préalablement décrites, l'étape 160 étant remplacée par une étape 161 dans laquelle, pour au moins une longueur d'onde $\lambda$, et pour une pluralité de distances de rétrodiffusion $D_n$, inférieures à 200 $\mu$m, on détermine le triplet ($\mu_a(\lambda)$, $\mu'_s(\lambda)$,g) présentant le moindre écart entre la réflectance $R_n(\lambda)$ mesurée, à la longueur d'onde $\lambda$, et une réflectance modélisée $R^{model}_{n,\mu a,\mu s',g}$ pour différentes valeurs du triplet ($\mu$a, $\mu$s′, g). Cette détermination peut être réalisée en minimisant un écart quadratique, selon l'expression :

$$\left(\mu_a(\lambda), \mu'_s(\lambda), g(\lambda)\right) = argmin_{\mu a, \mu s', g}\left(\sum_{n=1}^{N}\left(R^{model}_{n,\mu a,\mu s',g}(\lambda,) - R_n(\lambda)\right)^2\right)$$

où :

- N désigne le nombre de distances prises en compte. Dans le cas présent, N doit être au moins supérieur à 3 car trois grandeurs sont à estimer, à savoir $\mu_a$, $\mu_s$′ et g.
- $R^{model}_{n,\mu a,\mu s',g}$ est une fonction de réflectance modélisée, établie pour une distance de rétrodiffusion $D_n$, et pour différents triplets de valeurs $\mu_a$, $\mu$s′ et g, au cours d'une phase de calibration, comme précédemment décrit.

Cette étape 161 peut être avantageusement mise en œuvre itérativement, en fixant, par exemple alternativement, deux paramètres, sur les trois recherchés, soit à une valeur initiale arbitraire soit à une valeur déterminée lors d'une itération précédente. L'algorithme itératif peut alors converger vers un triplet de valeurs ($\mu$a,$\mu$s′,g)

Les inventeurs ont par ailleurs réalisé différents modèles, montrant que lorsque la distance de rétrodiffusion est faible, typiquement inférieure à 1 / $\mu_s$′, l'influence du coefficient d'absorption $\mu_a$ sur la réflectance est faible.

Tirant profit de ces constatations, il est proposé un troisième exemple de procédé, qui constitue un procédé préférentiel de détermination des propriétés optiques, plus robuste que le procédé précédent, et représenté sur la figure 5C.

Il comprend les étapes 110 à 150 telles que précédemment décrites, l'étape 120 comprenant l'acquisition :

- de deux signaux optiques de rétrodiffusion ($52_1$, $52_2$), dits signaux proches, émanant respectivement de la surface de l'échantillon à deux distances de rétrodiffusion $D_1$ et $D_2$ dites « proches », inférieures à 200 $\mu$m, ou, de façon plus générale, inférieures à $1/\mu$s′.

- de deux signaux optiques de rétrodiffusion, dits signaux éloignés ($52_3$, $52_4$), émanant respectivement de la surface de l'échantillon à deux distances de rétrodiffusion $D_3$ et $D_4$ dites « éloignées », supérieures auxdites distances proches ($D_1$, $D_2$) et de préférence supérieures à 200 $\mu$m, ou, de façon plus générale, supérieures à $1/\mu$s′, et encore de préférence supérieures à 500 $\mu$m.

L'étape 160 est remplacée par une étape 162, comprenant :

- Sous-Etape 162.1 : la détermination, pour au moins une longueur d'onde $\lambda$, ou pour au moins une bande spectrale, du coefficient de diffusion réduit $\mu_s$′($\lambda$) et du coefficient d'absorption $\mu_a(\lambda)$ par comparaison des réflectances $R_3$ et

$R_4$, mesurées aux distances de rétrodiffusion éloignées $D_3$ et $D_4$, avec des valeurs de réflectances modélisées $R_{3,\mu a,\mu s'}^{model}$, $R_{4,\mu a,\mu s'}^{model}$ de façon analogue à l'étape 160 précédemment décrite. Cette comparaison peut prendre la forme d'une minimisation d'un écart quadratique, selon l'expression :

$$\left(\mu_a(\lambda), \mu'_s(\lambda)\right) = argmin_{\left(\mu_a(\lambda),\mu'_s(\lambda)\right)} \left(\sum_{n=3}^{n=4} \left(R_{n,\mu a,\mu s'}^{model}(\lambda) - R_n(\lambda)\right)^2\right)$$

où $R_{n,\mu a,\mu s'}^{model}$ désigne une réflectance modélisée à la distance de rétrodiffusion $D_n$, pour un couple de valeurs $(\mu_a, \mu_s')$.

- Sous-étape 162.2 : la détermination, pour la même longueur d'onde, ou la même bande spectrale que lors de la sous-étape précédente, du coefficient de diffusion $\mu_s(\lambda)$ et du coefficient d'anisotropie g, par comparaison des réflectances $R_i$ et $R_2$, mesurées aux distances courtes $D_1$ et $D_2$, avec des valeurs de réflectances $R_{1,\mu s,g}^{model}$ et $R_{2,\mu s,g}^{model}$, modélisées en considérant respectivement les distances de rétrodiffusion $D_1$ et $D_2$, ainsi que différentes valeurs de $\mu_s$ et de g, de façon analogue à l'étape 160 précédemment décrite. Cette comparaison peut prendre la forme d'une minimisation d'un écart quadratique, selon l'expression :

$$(\mu_s(\lambda), g) = argmin_{(\mu s(\lambda),g)} \left(\sum_{n=1}^{n=2} \left(R_{n,\mu s,g}^{model}(\lambda) - R_n(\lambda)\right)^2\right)$$

où $R_{n,\mu s,g}^{model}$ désigne une réflectance modélisée à la distance de rétrodiffusion $D_n$, et un couple de valeurs $(\mu_s,g)$.

Cet algorithme est mis en œuvre en considérant la contrainte suivante :

$$\mu_s'(\lambda) = \mu_s(\lambda) (1-g(\lambda))$$

$\mu_s'(\lambda)$ étant déterminé lors de l'étape précédente 162.1.

Par ailleurs, le fait de déterminer $\mu_a$, lors de la première étape, permet, lors de la deuxième étape de sélectionner des valeurs de la réflectance modélisée $R_{n,\mu s,g}^{model}$ tenant compte de la valeur $\mu_a$ ainsi établie. On voit tout l'intérêt de combiner des mesures à grandes et à faibles distance de rétrodiffusion.

Lorsque la valeur du coefficient d'absorption est connue, ou fixée arbitrairement, l'étape 162.1 vise uniquement à déterminer $\mu_s'(\lambda)$ en prenant en compte la valeur dudit coefficient d'absorption. Dans ce cas, il n'est pas nécessaire de disposer de deux mesures $D_3$, $D_4$ à des distances éloignées. Une mesure à une seule distance ($D_3$ ou $D_4$) peut suffire.

Par ailleurs, un même signal rétrodiffusé peut être utilisé à la fois lors de la première étape 162.1 et lors de la deuxième étape 162.2. On améliore alors la précision de la mesure, en particulier lorsque le coefficient $\mu_s'$ n'est pas homogène selon la profondeur de l'échantillon.

Les procédés précédents ont été décrits en considérant un signal de réflectance $R_n$ établi, en appliquant une fonction de calibration *fcalib* à l'intensité d'un signal de rétrodiffusion $S_n$. D'une manière générale la fonction de calibration effectue un ajustement du signal de rétrodiffusion $S_n$ en prenant en compte des paramètres d'instruments, en particulier le bruit, l'efficacité, et éventuellement une normalisation par l'intensité du faisceau incident.

Lorsque le détecteur est pourvu d'une fonction spectrométrique, les procédés précédemment décrits peuvent être mises en œuvre pour une pluralité de longueurs d'onde ou de bandes spectrales différentes. Dans ce cas, on obtient les différentes propriétés optiques en fonction de la longueur d'onde ou de la bande spectrale considérée.

**Revendications**

1. Procédé de détermination d'une propriété optique ($\mu_a$, $\mu_s$, $\mu_s'$, g) d'un échantillon (50), comportant les étapes suivantes :

   - illumination d'une surface de l'échantillon à l'aide d'un faisceau lumineux (20), de façon à former, à la surface dudit échantillon, une zone élémentaire d'illumination (18), correspondant à la partie de ladite surface éclairée sur ledit échantillon,
   - détection de N signaux optiques ($52_1$, $52_2$, $52_3$, $52_4$, $52_n$), rétrodiffusés par l'échantillon, chaque signal optique émanant de la surface de l'échantillon, au niveau d'une zone élémentaire de détection ($28_n$), à une distance ($D_1$, $D_2$, $D_3$, $D_4$, $D_n$), dite distance de rétrodiffusion, de ladite zone élémentaire d'illumination, N étant un entier supérieur à 1, de manière à former autant de signaux détectés ($S_1$, $S_2$, $S_3$, $S_4$, $S_n$), chaque zone élémentaire de détection ($28_n$) étant séparée de ladite zone élémentaire d'illumination (18),
   - détermination d'au moins une propriété optique (p) de l'échantillon, par comparaison entre :

     - une fonction ($R_1$, $R_2$, $R_3$, $R_4$, $R_n$) de chaque signal ainsi détecté ($S_1$, $S_2$, $S_3$, $S_4$, $S_n$),
     - et une pluralité d'estimations de ladite fonction ($R_{1,p}^{model}$, $R_{2,p}^{model}$, $R_{3,p}^{model}$, $R_{4,p}^{model}$, $R_{n,p}^{model}$) chaque estimation étant réalisée en considérant une valeur prédéterminée de ladite propriété optique (p), le procédé étant **caractérisé en ce que**

   - lors de ladite étape de détection, au moins une distance de rétrodiffusion ($D_1$, $D_2$) séparant une zone élémentaire de détection ($28_1$, $28_2$) de ladite zone élémentaire d'illumination (18), est inférieure à 200 $\mu$m.

2. Procédé selon la revendication 1, dans lequel au moins une distance de rétrodiffusion ($D_1$, $D_2$,) est inférieure à 150 $\mu$m.

3. Procédé selon l'une des revendications 1 ou 2, dans lequel au moins deux distances de rétrodiffusion ($D_1$, $D_2$) sont inférieures à 200 $\mu$m.

4. Procédé selon l'une des revendications précédentes, dans lequel ladite propriété optique comprend :

   - un facteur d'anisotropie, représentant un angle moyen de diffusion,
   - un coefficient d'absorption,
   - un coefficient de diffusion,
   - un coefficient de diffusion réduit.

5. Procédé selon l'une des revendications précédentes, comprenant :

   - la détection d'au moins deux signaux optiques rétrodiffusés émanant de la surface de l'échantillon :

     - un signal rétrodiffusé proche ($52_1$, $52_2$), émis à une distance de rétrodiffusion dite proche ($D_1$, $D_2$), inférieure à 200 $\mu$m,
     - un signal rétrodiffusé éloigné ($52_3$, $52_4$), émis à une distance de rétrodiffusion dite éloignée ($D_3$, $D_4$), supérieure à ladite distance proche,

   - la détermination d'une première propriété optique ($\mu_s$, g) à partir dudit signal proche,
   - la détermination d'une deuxième propriété optique ($\mu_s'$, $\mu_a$), différente de la première propriété optique, à partir dudit signal rétrodiffusé éloigné.

6. Procédé selon la revendication 5, comprenant :

   - la détection d'au moins deux signaux optiques rétrodiffusés ($52_1$, $52_2$) proches, émanant de la surface de l'échantillon à deux distances de rétrodiffusion différentes ($D_1$, $D_2$), ces deux distances étant inférieures à 200 $\mu$m,
   - la détermination d'un coefficient de diffusion réduit ($\mu_s'$) à partir dudit signal optique éloigné ($S_3$, $S_4$),
   - puis la détermination d'un coefficient de diffusion ($\mu_s$) et d'un coefficient d'anisotropie de diffusion (g) à partir desdits deux signaux optiques rétrodiffusés proches ($52_1$, $52_2$), ainsi que du coefficient de diffusion réduit ($\mu_s'$) préalablement déterminé.

**7.** Procédé selon la revendication 6, comprenant :

- la détection d'au moins deux signaux optiques rétrodiffusés éloignés ($52_3$, $52_4$), émanant de la surface de l'échantillon à deux distances de rétrodiffusion différentes ($D_3$, $D_4$), les deux distances étant supérieures à 200 $\mu$m,
- la détermination d'uncoefficient d'absorption ($\mu_a$) à partir desdits signaux optiques rétrodiffusés éloignés.

**8.** Procédé selon l'une des revendications précédentes, dans lequel l'échantillon examiné est la peau d'un humain ou d'un animal.

**9.** Procédé selon l'une des revendications précédentes, dans lequel le signal optique rétrodiffusé est mesurée à une pluralité de longueurs d'ondes.

**10.** Procédé selon l'une des revendications précédentes, dans lequel la dite fonction du signal est la réflectance ($R_n$), représentant l'intensité du signal mesuré ($S_n$), correspondant à ladite distance de rétrodiffusion ($D_n$), relativement à l'intensité dudit faisceau lumineux (20).

**Patentansprüche**

**1.** Verfahren zum Bestimmen einer optischen Eigenschaft ($\mu_a$, $\mu_s$, $\mu_s'$, g) einer Probe (50), das die folgenden Schritte umfasst:

- Beleuchten einer Oberfläche der Probe mit Hilfe eines Lichtstrahls (20), um an der Oberfläche der Probe eine elementare Beleuchtungszone (18) zu bilden, die dem bestrahlten Oberflächenabschnitt auf der Probe entspricht,
- Erfassen von N optischen Signalen ($52_1$, $52_2$, $52_3$, $52_4$, $52_n$), die durch die Probe zurückgestreut werden, wobei jedes optische Signal mit einem Abstand ($D_1$, $D_2$, $D_3$, $D_4$, $D_n$), der als Rückstreuabstand bezeichnet wird, zu der elementaren Beleuchtungszone im Bereich einer elementaren Erfassungszone ($28_n$) von der Oberfläche der Probe ausgeht, wobei N eine ganze Zahl größer 1 ist, um ebenso viele erfasste Signale ($S_1$, $S_2$, $S_3$, $S_4$, $S_n$) zu bilden, wobei jede elementare Erfassungszone ($28_n$) von der elementaren Beleuchtungszone (18) getrennt ist,
- Bestimmen mindestens einer optischen Eigenschaft (p) der Probe durch Vergleichen von:

  - einer Funktion ($R_1$, $R_2$, $R_3$, $R_4$, $R_n$) jedes so erfassten Signals ($S_1$, $S_2$, $S_3$, $S_4$, $S_n$),
  - und einer Vielzahl von Berechnungen der Funktion ($R_{1,p}^{model}$, $R_{2,p}^{model}$, $R_{3,p}^{model}$, $R_{4,p}^{model}$ $R_{n,p}^{model}$, wobei jede Berechnung unter Berücksichtigung eines vorgegebenen Werts der optischen Eigenschaft (p) vorgenommen wird,

  wobei das Verfahren **dadurch gekennzeichnet ist, dass**

- während des Erfassungsschritts mindestens ein Rückstreuabstand ($D_1$, $D_2$), der eine elementare Erfassungszone ($28_1$, $28_2$) von der elementaren Beleuchtungszone (18) trennt, kleiner als 200 $\mu$m ist.

**2.** Verfahren nach Anspruch 1, wobei mindestens ein Rückstreuabstand ($D_1$, $D_2$) kleiner als 150 $\mu$m ist.

**3.** Verfahren nach einem der Ansprüche 1 oder 2, wobei mindestens zwei Rückstreuabstände ($D_1$, $D_2$) kleiner als 200 $\mu$m sind.

**4.** Verfahren nach einem der vorhergehenden Ansprüche, wobei die optische Eigenschaft Folgendes beinhaltet:

  - einen Anisotropiefaktor, der einen mittleren Streuwinkel darstellt,
  - einen Absorptionskoeffizienten,
  - einen Streukoeffizienten,
  - einen reduzierten Streukoeffizienten.

**5.** Verfahren nach einem der vorhergehenden Ansprüche, das Folgendes beinhaltet:

- das Erfassen von mindestens zwei zurückgestreuten optischen Signalen, die von der Oberfläche der Probe ausgehen:

- ■ eines nahen zurückgestreuten Signals ($52_1$, $52_2$), das mit einem Rückstreuabstand, der als nahe bezeichnet wird ($D_1$, $D_2$) und der kleiner als 200 $\mu$m ist, ausgesendet wird,
- ■ eines entfernten zurückgestreuten Signals ($52_3$, $52_4$), das mit einem Rückstreuabstand, der als entfernt bezeichnet wird ($D_3$, $D_4$) und der größer als der nahe Abstand ist, ausgesendet wird,

- das Bestimmen einer ersten optischen Eigenschaft ($\mu_s$, g) anhand des nahen Signals,
- das Bestimmen einer zweiten optischen Eigenschaft ($\mu_s'$, $\mu_a$), die sich von der ersten optischen Eigenschaft unterscheidet, anhand des entfernten zurückgestreuten Signals.

6. Verfahren nach Anspruch 5, das Folgendes beinhaltet:

- das Erfassen von mindestens zwei nahen zurückgestreuten optischen Signalen ($52_1$, $52_2$), die mit zwei unterschiedlichen Rückstreuabständen ($D_1$, $D_2$) von der Oberfläche der Probe ausgehen, wobei diese zwei Abstände kleiner als 200 $\mu$m sind,
- das Bestimmen eines reduzierten Streukoeffizienten ($\mu_s'$) anhand des entfernten optischen Signals ($S_3$, $S_4$),
- dann das Bestimmen eines Streukoeffizienten ($\mu_s$) und eines Streuanisotropiekoeffizienten (g) anhand der zwei nahen zurückgestreuten optischen Signale ($52_1$, $52_2$) sowie des zuvor bestimmten reduzierten Streukoeffizienten ($\mu_s'$).

7. Verfahren nach Anspruch 6, das Folgendes beinhaltet:

- das Erfassen von mindestens zwei entfernten zurückgestreuten optischen Signalen ($52_3$, $52_4$), die mit zwei unterschiedlichen Rückstreuabständen ($D_3$, $D_4$) von der Oberfläche der Probe ausgehen, wobei die zwei Abstände größer als 200 $\mu$m sind,
- das Bestimmen eines Absorptionskoeffizienten ($\mu_a$) anhand der entfernten zurückgestreuten optischen Signale.

8. Verfahren nach einem der vorhergehenden Ansprüche, wobei die untersuchte Probe die Haut eines Menschen oder eines Tieres ist.

9. Verfahren nach einem der vorhergehenden Ansprüche, wobei das zurückgestreute optische Signal mit einer Vielzahl von Wellenlängen gemessen wird.

10. Verfahren nach einem der vorhergehenden Ansprüche, wobei die Funktion des Signals das Reflexionsvermögen ($R_n$) ist, das die Stärke des gemessenen Signals ($S_n$), das dem Rückstreuabstand ($D_n$) entspricht, relativ zu der Stärke des Lichtstrahls (20) darstellt.

**Claims**

1. Method for determining an optical property ($\mu_a$, $\mu_s$, $\mu_s'$, g) of a sample (50), comprising the following steps:

- illumination of a surface of the sample with the aid of a light beam (20), so as to form, on the surface of the said sample, an elementary illumination zone (18), corresponding to the part of the said surface illuminated on the said sample,
- detection of N optical signals ($52_1$, $52_2$, $52_3$, $52_4$, $52_n$), backscattered by the sample, each optical signal emanating from the surface of the sample, at the level of an elementary detection zone ($28_n$), at a distance ($D_1$, $D_2$, $D_3$, $D_4$, $D_n$), termed the backscattering distance, from the said elementary illumination zone, N being an integer greater than 1, so as to form as many detected signals ($S_1$, $S_2$, $S_3$, $S_4$, $S_n$), each elementary detection zone ($28_n$) being separated from the said elementary illumination zone (18),
- determination of at least one optical property (p) of the sample, by comparison between:

  - ■ a function ($R_1$, $R_2$, $R_3$, $R_4$, $R_n$) of each signal thus detected ($S_i$, $S_2$, $S_3$, $S_4$, $S_n$),
  - ■ and a plurality of estimations of the said function ($R_{1,p}^{model}$, $R_{2,p}^{model}$, $R_{3,p}^{model}$, $R_{4,p}^{model}$, $R_{n,p}^{model}$), each estimation being carried out by considering a predetermined value of the said optical property (p), the method being **characterized in that**

- during the said detection step, at least one backscattering distance ($D_1$, $D_2$) separating an elementary detection zone ($28_1$, $28_2$) from the said elementary illumination zone (18), is less than 200 $\mu$m.

2.  Method according to Claim 1, in which at least one backscattering distance ($D_1$, $D_2$,) is less than 150 $\mu$m.

3.  Method according to either of Claims 1 and 2, in which at least two backscattering distances ($D_1$, $D_2$) are less than 200 $\mu$m.

4.  Method according to one of the preceding claims, in which the said optical property comprises:

    - an anisotropy factor, representing a mean angle of scattering,
    - an absorption coefficient,
    - a scattering coefficient,
    - a reduced scattering coefficient.

5.  Method according to one of the preceding claims, comprising:

    - the detection of at least two backscattered optical signals emanating from the surface of the sample:

        - a near backscattered signal ($52_1$, $52_2$), emitted at a so-called near backscattering distance ($D_1$, $D_2$), less than 200 $\mu$m,
        - a far backscattered signal ($52_3$, $52_4$), emitted at a so-called far backscattered distance ($D_3$, $D_4$), greater than the said near distance,

    - the determination of a first optical property ($\mu_s$, g) on the basis of the said near signal,
    - the determination of a second optical property ($\mu_s$', $\mu_a$), different from the first optical property, on the basis of the said far backscattered signal.

6.  Method according to Claim 5, comprising:

    - the detection of at least two near backscattered optical signals ($52_1$, $52_2$) emanating from the surface of the sample at two different backscattering distances ($D_1$, $D_2$), these two distances being less than 200 $\mu$m,
    - the determination of a reduced scattering coefficient ($\mu_s$') on the basis of the said far optical signal ($S_3$, $S_4$),
    - then the determination of a scattering coefficient ($\mu_s$) and of a scattering anisotropy coefficient (g) on the basis of the said two near backscattered optical signals ($52_1$, $52_2$), as well as of the previously determined reduced scattering coefficient ($\mu_s$').

7.  Method according to Claim 6, comprising:

    - the detection of at least two far backscattered optical signals ($52_3$, $52_4$), emanating from the surface of the sample at two different backscattering distances ($D_3$, $D_4$), the two distances being greater than 200 $\mu$m,
    - the determination of an absorption coefficient ($\mu_a$) on the basis of the said far backscattered optical signals.

8.  Method according to one of the preceding claims, in which the sample examined is the skin of a human or of an animal.

9.  Method according to one of the preceding claims, in which the backscattered optical signal is measured at a plurality of wavelengths.

10. Method according to one of the preceding claims, in which the said function of the signal is the reflectance ($R_n$), representing the intensity of the measured signal ($S_n$), corresponding to the said backscattering distance ($D_n$), relative to the intensity of the said light beam (20).

**Fig.1**

**Fig.2**

**Fig.3A**

**Fig.3B**

20  $52_1$  $52_2$  $52_3$  $52_4$

18  $28_1$  $28_2$  $28_3$  $28_4$

$z_1$  $z_2$  $z_3$  $z_4$

$F_1$  $F_2$  $F_3$  $F_4$

**Fig.4**

| 110 | 110 | 110 |
| 120 | 120 | 120 |
| 130 | 130 | 130 |
| 140 | 140 | 140 |
| 150 | 150 | 150 |
| 160 | 161 | 162.1 / 162 / 162.2 |

**Fig.5A**  **Fig.5B**

**Fig.5C**

**Fig.6**

**Fig. 7A**

**Fig. 7B**

**Fig. 7C**

Fig.7D

**EP 3 054 282 B1**

## RÉFÉRENCES CITÉES DANS LA DESCRIPTION

**Documents brevets cités dans la description**

- US 2003191398 A
- US 20120302892 A
- WO 2011115627 A
- EP 2762064 A